# EUROPEAN PATENT APPLICATION

(11) **EP 1 859 802 A2**
(43) Date of publication of application: **28.11.2007**
(21) Application number: 07114501.5
(22) Date of filing: 03.09.2004
(51) Int. Cl.: A61K 31/495, A61K 31/5575, A61P 27/02

(54) **Compositions comprising benzo(G)quinoline derivates and prostaglandin derivates**

(30) Priority: 05.09.2003 GB 0320869; 10.10.2003 GB 0323828
(62) Divisional of application: 04764818.3
(71) Applicant: Novartis AG, 4056 Basel (CH); Novartis Pharma GmbH, 1230 Vienna (AT)
(72) Inventor: Lambrou, George N., 67100, Strasbourg (FR); Latour, Elisabeth Jeanne, 68870, Bartenheim-La Chaussée (FR); Reinhart, Thomas, 79540, Lörrach (DE)
(74) Representative: von Sprecher, Georg

(57) **Abstract**

The present invention relates to a fixed combination of a benzo[g]quinoline derivative and a prostaglandin derivative, and their use as pharmaceuticals, especially for healing glaucoma.

## Description

The present invention relates to a fixed combination of a benzo[g]quinoline derivative and a prostaglandin derivative, their use as pharmaceuticals, in particular with their synergistic efficacy in lowering elevated levels of the intraocular pressure (IOP).

As used herein, a benzo[g]quinoline derivative pertains to a compound of formula (I), 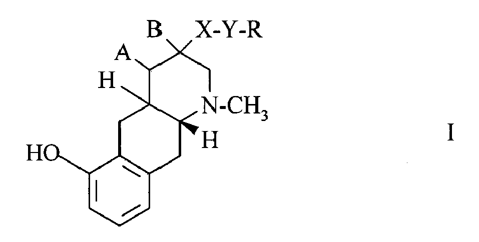 wherein
- A and B: are each H or form together an additional bond,
- X: is CH₂ or CO,
- Y: is O, S, NR₁ (R₁ being H or lower alkyl), CH₂ or O-CH₂, and
- R: is of formula (a), (b), (c), (d), (e), (f), (g), (h) or (i), 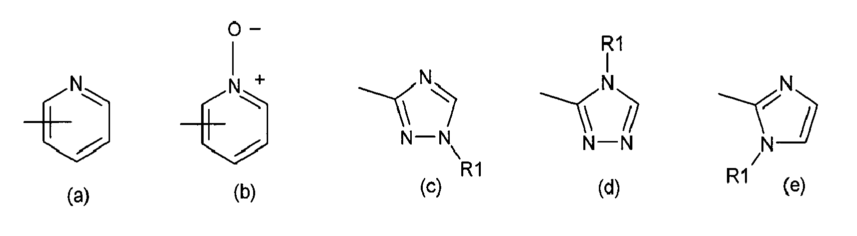 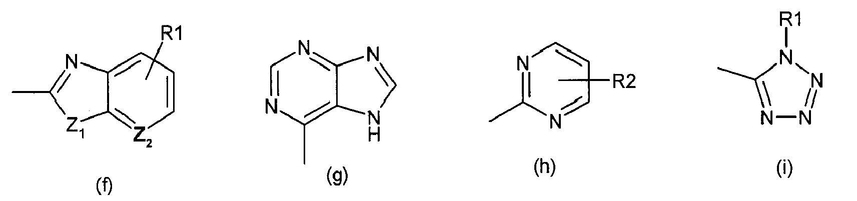
R₁ being H or lower alkyl,
R₂ being H, lower alkyl, or thienyl, e.g. 2-thienyl,
Z₁ being O or S,
Z₂ being CH or N,
in free base or acid addition salt form.

The above-defined lower alkyl groups preferably represent methyl, ethyl, propyl and iso-propyl, more preferably methyl.

As used herein, the prefix "lower" denotes a radical having up to and including a maximum of 7, especially up to and including a maximum of 4 carbon atoms, the radicals in question being either linear or branched with single or multiple branching.

When A and B are each H, the X-Y-R substituent preferably presents the configuration 3R.

Acid addition salts may be produced from the free bases in known manner, and vice versa. Suitable acid addition salts for use in accordance with the present invention include for example the hydrochloride.

Preferably in a compound of formula (I),
- A and B: are each H or form together an additional bond,
- X: is CH₂
- Y: is S, and
- R: is of formula (e), (f), or (h), 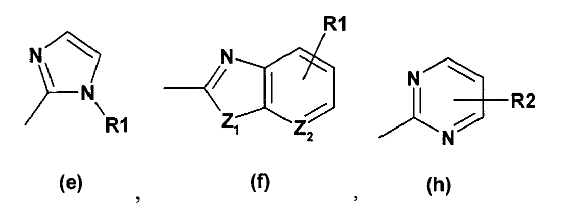
wherein
R₁ denotes H or lower alkyl,
Z₁ being S,
Z₂ being CH or N, and
R₂ denotes H, or lower alkyl.

A preferred benzo[g]quinoline is selected from the group consisting of:

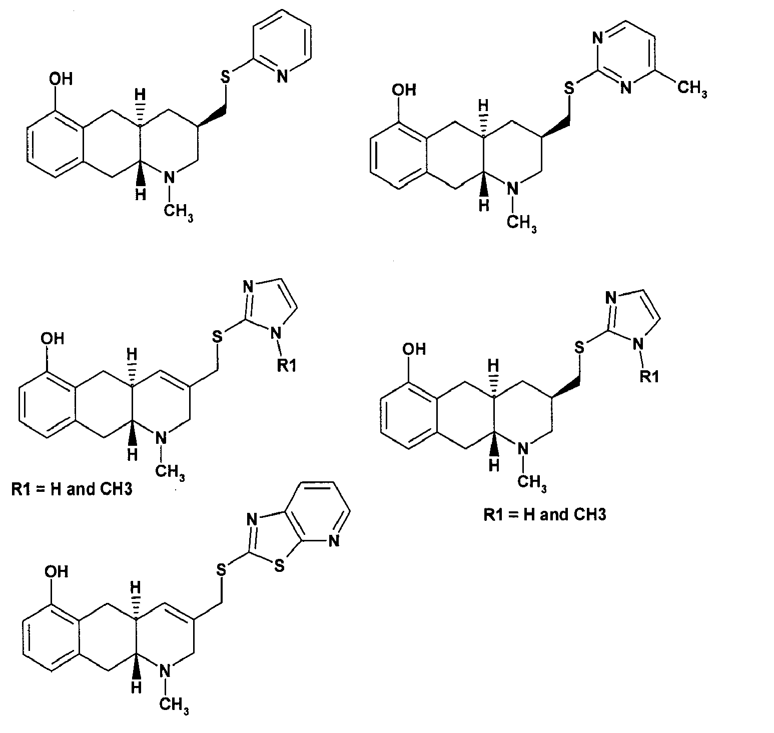

The benzo[g]quinoline compounds disclosed above are described and / or are obtainable as described in EP 77754, WO 98/01444, WO 00/37571, WO 03/006458 and WO 03/074511.

As used herein, a prostaglandin derivative pertains to a compound of formula II, 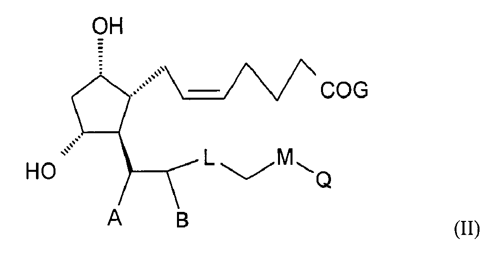 wherein G denotes OH, lower alkoxy, amino-lower-alkyl,,
A and B are each H or form together an additional bond,
L is CO, CHOR₁ (R₁ being H or lower alkyl),
M is O, S, (CH₂)ₙ, (n being from 1 to 6),
Q is H or Ar, Ar being preferably phenyl which is unsubstituted or substituted one or more times by halogen, hydroxy, lower alkoxy, cyano, halo-lower-alkyl, e.g. trihalomethyl, in particular trifluoromethyl.

A preferred prostaglandin derivative is selected from the group consisting of:

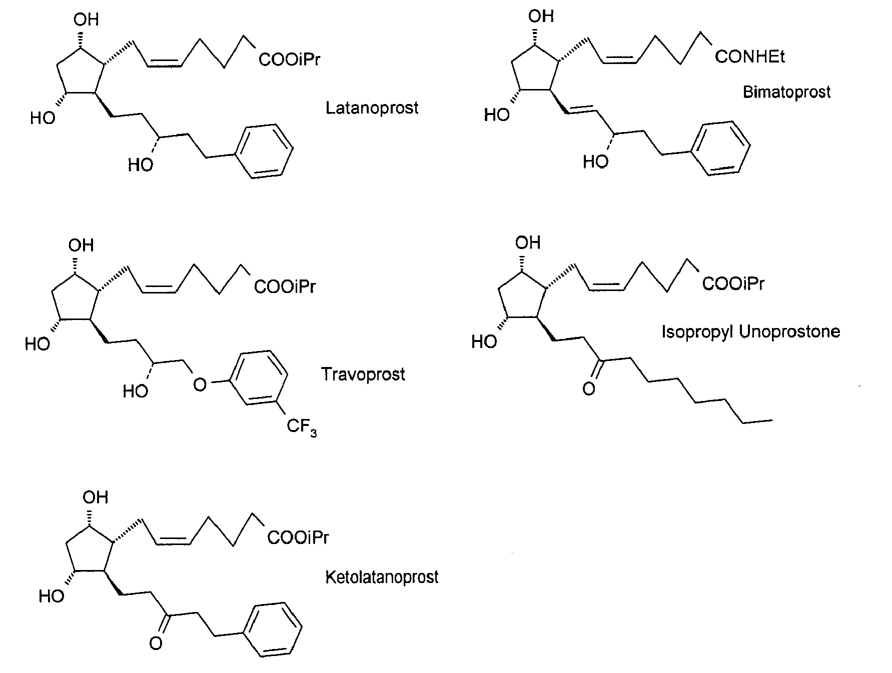

The above preferred prostaglandins are described in the art. Hence should the chemical structure deviate from its original, or should the above graphical description contain an error, then the above should be construed in accordance to the generic name provided.

In particular in a combination of the present invention a prostaglandin is selected from Latanoprost, Travaprost, Bimatoprost and Ketolatanoprost, more preferably from Latanoprost and Travaprost, and in particular preferably from Latanoprost.

The combination of a compound of formula I with a compound of formula II and their physiologically acceptable acid addition salts, referred to hereinafter as an agent of the invention, exhibit valuable pharmacological properties in animal tests and are therefore useful as pharmaceuticals.

A preferred combination of a benzo[g]quinoline with Latanoprost is:

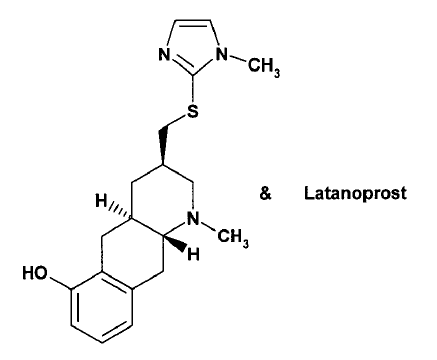

Another preferred combination of a benzo[g]quinoline with Latanoprost is:

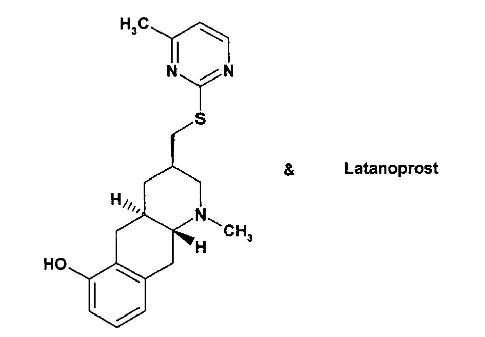

Another preferred combination of a benzo[g]quinoline with Latanoprost is::

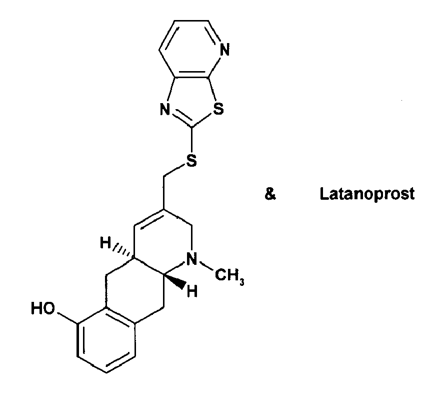

In particular, when an agent of the present invention is acidic, salts may be prepared from pharmaceutically acceptable non-toxic bases. Salts derived from all stable forms of inorganic bases include aluminum, ammonium, calcium, copper, iron, lithium, magnesium, manganese, potassium, sodium, zinc, etc. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines and basic ion-exchange resins such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, isopropylamine, lysine, methylglucosamine, morpholine, piperazine, piperidine, polyamine resins, procaine, purine, theobromine, triethylamine, trimethylamine, tripropylamine, etc.

When an agent of the present invention is basic, salts may be prepared from pharmaceutically acceptable non-toxic acids. Such acids include acetic, benzenesulfonic, benzoic, camphorsulfonic, citric, ethanesulfonic, fumaric, gluconic, glutamic, hydrobromic, hydrochloric, lactic, maleic, mandelic, methanesulfonic, mucic, nitric, pamoic, pantothenic, phosphoric, succinic, sulfuric, tartaric, p-toluenesulfonic, etc. Particularly preferred are citric, hydrobromic, maleic, phosphoric, sulfuric, fumaric and tartaric acids. Base salts also include ammonium, alkali metal, and alkaline earth metal salts, salts with organic bases, such as dicyclohexylamine salts, and salts with amino acids such as arginine and lysine. Also, basic nitrogen-containing groups can be quaternized with such agents as lower alkyl halides, such as methyl chloride, dialkyl sulfates, such as dimethyl, sulfates, long chain halides such as stearyl chlorides, and aralkyl halides, such as benzyl chlorides.

As used herein, a combination of a compound of formula (I) with a compound of formula (II) means that at least one compound of formula (I) is combined with at least one compound of formula (II).

As used herein, aryl stands for an aromatic moiety having from 6 to 14 carbon atoms, and is for example, phenyl or naphthyl that is unsubstituted or substituted by lower alkyl, lower alkoxy, hydroxy, lower alkoxycarbonyl, carboxy, carbamoyl, sulfamoyl, lower alkanoyl, halogen and/or by trifluoromethyl.
Aryl as used herein stands also for an unsubstituted or substituted heteroaromatic radical optionally partially hydrogenated, 5- or 6-membered monocyclic heteroaryl or bicyclic heteroaryl composed of 5- or 6-membered rings, such as corresponding furyl, lower alkylfuryl, for example 4-methylfur-2-yl, thienyl, imidazolyl, for example imidazol-4-yl, oxazolyl, carboxy-lower alkyl(oxo)oxazolyl, for example 2,5-dihydro-3-oxo-1,2-oxazolyl, thiazolyl, dihydrothiazolyl, for example 4,5-dihydrothiazolyl, carboxy-lower alkylthiazolyl, for example 4-carboxymethylthiazolyl, lower alkoxycarbonyl-lower alkylthiazolyl, for example 4-methoxycarbonylmethylthiazolyl or 4-ethoxycarbonyl-methylthiazolyl, tetrazolyl, pyridyl, pyrazinyl, indolyl, for example indol-3-yl, quinolinyl, for example quinolin-4-yl, benzazepinyl or carboxy-lower alkyl-2,3,4,5-tetrahydro-1H-1-benzazepino, for example 1-carboxymethyl-2,3,4,5-tetrahydro-1H-1-benzazepino.
Preferably, aryl is phenyl, pyridyl, thienyl or naphthyl that is unsubstituted or substituted by lower alkyl, lower alkoxy, hydroxy, carboxy, carbamoyl, sulfamoyl, lower alkanoyl, halogen and/or by trifluoromethyl.

Lower alkenyl is, for example, C₂-C₇alkenyl, such as vinyl, 1,3-prop-2-enyl, 1,2-prop-2-enyl, 1,4-but-2-enyl, 1,2-but-3-enyl, 1,2-pent-4-enyl, 1,2-hex-4-enyl or 1,2-hex-5-enyl. Preferred is vinyl, 1,3-prop-2-enyl, 1,2-prop-2-enyl or 1,4-but-2-enyl, more preferred is vinyl, 1,3-prop-2-enyl, or 1,2-prop-2-enyl.

Lower alkinyl is, for example, C₂-C₇ alkinyl, such as ethinyl, 1-propin-1-yl, 1-propin-3-yl, 1-butin-1-yl, 1-butin-4-yl and the like. Preferred is ethinyl, 1-propin-1-yl, or 1-propin-3-yl. More preferred is ethinyl or 1-propin-3-yl.

Lower alkoxy is, for example, C₁-C₇alkoxy, preferably C₁-C₅alkoxy, such as methoxy, ethoxy, propyloxy, isopropyloxy or butyloxy, but may also be isobutyloxy, sec-butyloxy, tert-butyloxy or a pentyloxy, hexyloxy or heptyloxy group.

As used herein halogen is preferably fluorine, chlorine, bromine, or iodine, more preferably fluorine, or chlorine, highly preferably fluorine.

In a combination of two compounds, the two compounds of formula (I) and (II) are typically present in a ratio of from 50:1 up to 1:50. Examples of such ratios are 20:1, 10:1, 5:1, 2:1,1:1, 1:2, 1:3, 1:5, 1:7, 1:10, 1:20, and 1:50. Said ratio is in weight percent of the total amount of active ingredients.

A surprising efficacy of the agents of the present invention is the synergistic IOP-lowering efficacy.

### Biological Part

In particular, the agents according to the invention induce a decrease on the intraocular pressure (IOP) in humans. For example, in subjects with bilateral primary open-angle glaucoma or ocular hypertension, the agents, when applied topically to the eye as for example an 0.1% ophthalmic solution (e.g. one drop b.i.d. in each eye), typically lowered IOP by about 20% relative to baseline. The combination of the above actives (benzo[g]quinoline-derivative and prostaglandin-derivative) exhibit a synergistic IOP-lowering efficacy.

The agents according to the invention are therefore useful in the treatment of glaucoma.

For the above mentioned indication, the appropriate dosage will of course vary depending upon, for example, the compounds employed, the host, the mode of administration and the severity of the condition being treated.

The agents of the invention may be administered in free form or in pharmaceutically acceptable salt form. Such salts may be prepared in conventional manner and exhibit the same order of activity as the free compounds.

Accordingly the present invention provides an agent of the invention for use as a pharmaceutical, e.g. in the treatment of glaucoma.

Another surprising finding of the agents according to the invention is the improvement of the chorio-retinal and optic nerve blood flow. Decrease of the optic nerve head blood perfusion is a possible factor in the pathogenesis of normal tension glaucoma (NTG), visual field damage, excavation of the optic nerve head. Hence, the agents according to the invention are also useful in the treatment of NTG and to prevent visual field damage and/or excavation of the optic nerve head. In a preferred aspect the agents according to the invention are useful in the treatment of NTG.

The present invention furthermore provides a pharmaceutical composition comprising an agent of the invention in association with at least one pharmaceutically acceptable carrier. Such compositions may be formulated in conventional manner.

Agents according to the invention may be administered by any conventional route, for example parenterally e.g. in form of injectable solutions or suspensions, or enterally, preferably orally, e.g. in the form of tablets or capsules, or topically, e.g. as a solution, ointment, or gel.

More preferably, they are applied topically to the eye in ca. 0.0001 to 1 % ophthalmological solutions.

The ophthalmic vehicle is such that the compound is maintained in contact with the ocular surface for a sufficient time period to allow the compound to penetrate the corneal and internal regions of the eye.

The pharmaceutically acceptable ophthalmic vehicle may be e.g. an ointment, vegetable oil, or an encapsulating material.

In still a further aspect the present invention provides a method for the treatment of glaucoma in a subject in need of such treatment, which comprises administering to such subject a therapeutically effective amount of an agent of the invention.

Any suitable route of administration may be employed for providing a mammal, especially a human, with an effective dosage of an agent of the invention. For example, oral, rectal, topical, parenteral, ocular, pulmonary, nasal, etc. routes may be employed. Dosage forms include tablets, troches, dispersions, suspensions, solutions, capsules, creams, ointments, aerosols, and the like.

A carrier may take a wide variety of forms depending on the nature of the preparation desired for administration, i.e., oral, parenteral, etc. In preparing oral dosage forms, any of the usual pharmaceutical media may be used, such as water, glycols, oils, alcohols, flavoring agents, preservatives, coloring agents, and the like in the case of oral liquid preparations (e.g., suspensions, elixirs, and solutions); or carriers such as starches, sugars, microcrystalline cellulose, diluents, granulating agents, lubricants, binders, disintegrating agents, etc. in the case of oral solid preparations such as powders, capsules, and tablets. Solid oral preparations are preferred over liquid oral preparations. Because of their ease of administration, tablets and capsules are the preferred oral dosage unit form. If desired, capsules may be coated by standard aqueous or non-aqueous techniques.

Pharmaceutical compositions of the present invention suitable for oral administration may be prepared as discrete units such as capsules, cachets, or tablets each containing a predetermined amount of the active ingredient in powder or granular form or as a solution or suspension in an aqueous or nonaqueous liquid or in an oil-in-water or water-in-oil emulsion. Such compositions may be prepared by any of the methods known in the art of pharmacy. In general, the compositions are prepared by uniformly and intimately admixing the active ingredient with liquid carriers, finely divided solid carriers, or both and then, if necessary, shaping the product into the desired form.

For example, a tablet may be prepared by compression or molding, optionally with one or more accessory ingredients. Compressed tablets may be prepared by compressing in a suitable machine the active ingredient in a free-flowing form such as powder or granule optionally mixed with a binder, lubricant, inert diluent, or surface active or dispersing agent. Molded tablets may be made by molding in a suitable machine, a mixture of the powdered compound moistened with an inert liquid diluent.
Ophthalmic inserts may be prepared from compression molded films e.g. prepared on a Carver Press by subjecting the powdered mixture of active ingredient and HPC to a compression force of 12,000 lb. (gauge) at 149°C for 1-4 min. In an example, the film is cooled under pressure by having cold water circulate in the platen. The inserts are then individually cut from the film with a rod-shaped punch. Each insert is placed in a vial, which is then placed in a humidity cabinet (88% relative humidity at 30°C) for 2-4 days. After removal from the cabinet, the vials are capped and then autoclaved at 121°C for 0.5 hr.

Where the combination is a topical composition, it may contain topically acceptable excipients or additives known to the person skilled in the art, for example ophthalmic carriers, isotonising agents also called tonicity enhancers, buffers, preservatives, complexing agents, solubilizers and other non-toxic excipients.

Carriers for liquid pharmaceutical, in particular ophthalmic administration are for example water, mixtures of water and water-miscible solvents, such as C₁- to C₇-alkanols, vegetable oils or mineral oils comprising from 0.5 to 5% by weight hydroxyethylcellulose, ethyl oleate, carboxymethyl-cellulose, polyvinyl-pyrrolidone and other non-toxic water-soluble polymers for ophthalmic uses, such as, for example, cellulose derivatives, such as methylcellulose, alkali metal salts of carboxymethylcellulose, hydroxymethylcellulose, hydroxyethylcellulose, methylhydroxypropyl-cellulose, hydroxypropylcellulose, chitosan and scleroglucan, polysaccharides such as hyaluronic acid, acrylates or methacrylates, such as salts of polyacrylic acid or ethyl acrylate, polyacrylamides, natural products, such as gelatin, alginates, pectins, tragacanth, karaya gum, xanthan gum, carrageenin, agar and acacia, starch derivatives, such as starch acetate and hydroxypropyl starch, and also other synthetic products, such as poloxamers, e.g. Poloxamer F127, polyvinyl alcohol, polyvinylpyrrolidone, polyvinyl methyl ether, polyethylene oxide, preferably cross-linked polyacrylic acid, such as neutral Carbopol, or mixtures of those polymers. Preferred carriers are water, cellulose derivatives, such as methylcellulose, alkali metal salts of carboxymethylcellulose, hydroxymethylcellulose, hydroxyethylcellulose, methylhydroxypropylcellulose and hydroxypropylcellulose, neutral Carbopol, or mixtures thereof. The concentration of the carrier is, for example, from 0.1 to 100000 times the concentration of an active ingredient.

Solubilizers may also be used in an ophthalmic composition of the present invention and are, for example, a cyclodextrin, tyloxapol, fatty acid glycerol polyethylene glycol esters, fatty acid polyethylene glycol esters, polyethylene glycols, glycerol ethers, polysorbate 20, polysorbate 80 or mixtures of those compounds. A specific example of an especially preferred solubilizer is a reaction product of castor oil and ethylene oxide, for example the commercial products Cremophor EL^{®}or Cremophor RH 40^{®}. Reaction products of castor oil and ethylene oxide have proved to be particularly good solubilizers that are tolerated extremely well by the eye. Other preferred solubilizers are cyclodextrins and tyloxapol. The concentration used depends especially on the concentration of the active ingredient. The amount added is typically sufficient to solubilize the active ingredient. For example, the concentration of the solubilizer is from 0.1 to 5000 times the concentration of an active ingredient.

Examples of buffers are acetate, ascorbate, borate, hydrogen carbonate / carbonate, citrate, gluconate, lactate, phosphate, propionate and TRIS (tromethamine) buffers. Tromethamine and borate buffer are preferred buffers. The amount of a buffer added is, for example, that necessary to ensure and maintain a physiologically tolerable pH range. The pH range is typically in the range of from 5 to 9, preferably from 6 to 8.5 and more preferably from 6.5 to 8.2.

Tonicity enhancing agents are, for example, ionic compounds, such as alkali metal or alkaline earth metal halides, such as, for example, CaCl₂, KBr, KCl, LiCl, NaI, NaBr or NaCl, Na₂SO₄ or boric acid. Non-ionic tonicity enhancing agents are, for example, urea, glycerol, sorbitol, mannitol, propylene glycol, or dextrose. For example, sufficient tonicity enhancing agent is added to impart to the ready-for-use ophthalmic composition an osmolality of approximately from 50 to 1000 mOsmol, preferred from 100 to 400 mOsmol, more preferred from 200 to 400 mOsmol and even more preferred from 250 to 350 mOsmol.

Examples of preservatives are quaternary ammonium salts such as e.g. sepazonium chloride, cetyltrimethylammonium bromide (cetrimide), cetylpyridinium chloride, benzoxonium chloride, benzethonium chloride, domiphen bromide (Bradosol^{®}) or benzalkonium chloride, alkyl-mercury salts of thiosalicylic acid, such as, for example, thiomersal, phenylmercuric nitrate, phenylmercuric acetate or phenylmercuric borate, parabens, such as, for example, methylparaben or propylparaben, alcohols, such as, for example, chlorobutanol, benzyl alcohol or phenyl ethanol, guanidine derivatives, such as, for example, chlorohexidine or polyhexamethylene biguanide, sodium perborate, Germal^{®}II or sorbic acid. Preferred preservatives are quaternary ammonium salts, alkyl-mercury salts and parabens. Where appropriate, a sufficient amount of preservative is added to the ophthalmic composition to ensure protection against secondary contaminations during use caused by bacteria and fungi.

An ophthalmic composition may further comprise other non-toxic excipients, such as, for example, emulsifiers, wetting agents or fillers, such as, for example, the polyethylene glycols designated 200, 300, 400 and 600, or Carbowax designated 1000, 1500, 4000, 6000 and 10000. Other excipients that may be used if desired are listed below but they are not intended to limit in any way the scope of the possible excipients. They are especially complexing agents, such as disodium-EDTA or EDTA, antioxidants, such as ascorbic acid, acetylcysteine, cysteine, sodium hydrogen sulfite, butyl-hydroxyanisole, butyl-hydroxytoluene or alpha-tocopherol acetate; stabilizers, such thiourea, thiosorbitol, sodium dioctyl sulfosuccinate or monothioglycerol; or other excipients, such as, for example, lauric acid sorbitol ester, triethanol amine oleate or palmitic acid ester. Preferred exipients are complexing agents, such as disodium-EDTA. The amount and type of excipient added is in accordance with the particular requirements and is generally in the range of from approximately 0.0001 to approximately 90% by weight.

### Galenic examples

In the subsequent examples I to V, the benzo[g]quinoline derivative is compound of formula III, 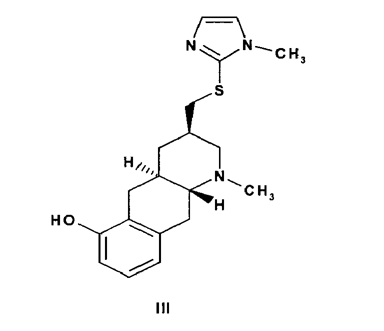 and the prostaglandin derivative is Latanoprost.

### Example I :

### Pharmaceutical composition for oral dosage form : tablet

| | |
|---|---|
| Benzo[g]quinoline derivative tablet) | range = 0.1 mg - 200 mg / per dosage (e.g. per |
| Prostaglandin derivative tablet) | range = 0.005 mg - 100 mg / per dosage (e.g. per |
| Maize Starch | 20.30 (% w/w) |
| Aerosil | 0.80 (% w/w) |
| Magnesium stearate | 0.70 (% w/w) |
| Lactose | ad to 100.00 (% w/w) |

### Example II :

### Pharmaceutical composition for liquid dosage form : parenteral administration (% w/w)

| | |
|---|---|
| Benzo[g]quinoline derivative | 0.005% - 2.0% |
| Prostaglandin derivative | 0.0005% - 0.5% |
| Polysorbate 20 | 0.2 |
| EDTA | 0.05 |
| Sodium chloride | ad to 280 mOsm/Kg |
| Phosphate buffer | ad to pH 7.4 |
| Water for injections | ad to 100.00 |

### Example III:

### Pharmaceutical composition for liquid dosage form : eye drop (% w/w)

| | |
|---|---|
| Benzo[g]quinoline derivative | 0.005% - 2.0% |
| Prostaglandin derivative | 0.0005% - 0.5% |
| Propyleneglycol | 2.00 |
| Benzalkonium chloride | 0.01 |
| EDTA | 0.10 |
| Phosphate buffer | ad to pH 6.8 |
| Sorbitol | ad to 300 mOsm/Kg |
| Water for injections | ad to 100.00 |

### Example IV :

### Pharmaceutical composition for topical administration : ophthalmic ointment (% w/w)

| | |
|---|---|
| Benzo[g]quinoline derivative | 0.005% - 2.0% |
| Prostaglandin derivative | 0.0005% - 0.5% |
| Phenyl Ethanol | 0.50 |
| Liquid Paraffin | 10.00 |
| White Petrolatum | ad to 100.00 |

### Example V :

### Pharmaceutical composition for topical administration : ophthalmic gel (% w/w)

| | |
|---|---|
| Benzo[g]quinoline derivative | 0.005% - 2.0% |
| Prostaglandin derivative | 0.0005% - 0.5% |
| Cremophor EL | 4.00 |
| Carbopol | 0.15 |
| Benzalkonium chloride | 0.01 |
| Tromethamine | ad to pH 6.8 |
| Propyleneglycol | ad to 300 mOsm/Kg |
| Water for injection | ad to 100.00 |

As used herein, the terms "treatment" or "treat" refer to both prophylactic or preventive treatment as well as curative or disease-modifying treatment, including treatment of patients at risk of contracting the disease or suspected to have contracted the disease as well as patients who are ill or have been diagnosed as suffering from a disease or medical condition.

The invention also pertains to a method to treat and or prevent glaucoma in a subject having in particular but not exclusively elevated levels of IOP, which method comprises the administration of a medicament to said patient, which medicament comprises a benzo[g]quinoline derivative and a prostaglandin derivative.

Other embodiments may be described in the independent and dependant claims only, but shall equally be part of the description of the present invention.

## Claims

1. A combination comprising a benzo[g]quinoline derivative and/or a pharmaceutically acceptable salt thereof, and a prostaglandin derivative and/or a pharmaceutically acceptable salt thereof.

2. A combination of claim 1, wherein said benzo[g]quinoline is a compound of formula (I) and/or a pharmaceutically acceptable salt thereof 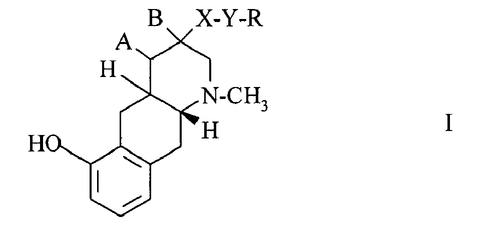 wherein
A and B are each H or form together an additional bond,
X is CH₂ or CO,
Y is O, S, NR₁ (R₁ being H or lower alkyl), CH₂ or O-CH₂, and
R is of formula (a), (b), (c), (d), (e), (f), (g), (h) or (i). 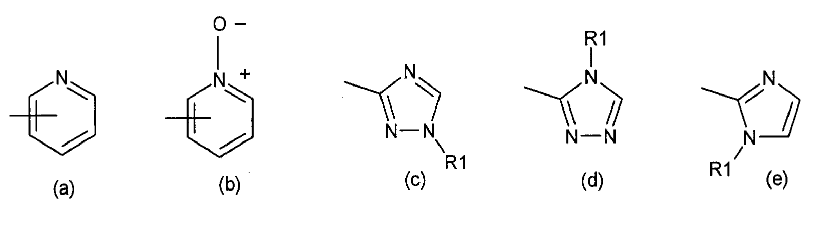 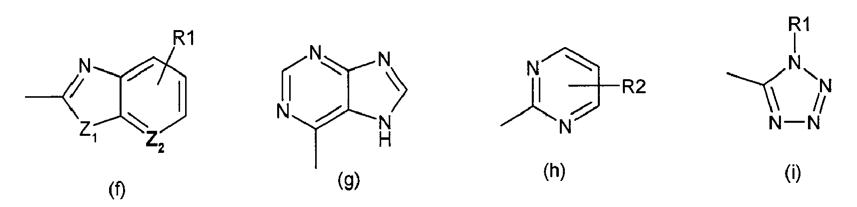
R₁ being H or lower alkyl,
R₂ being H, lower alkyl, or thienyl, e.g. 2-thienyl,
Z₁ being O or S,
Z₂ being CH or N,
in free base or acid addition salt form,
and wherein said prostaglandin derivative is a compound of formula (II) and/or a pharmaceutically acceptable salt thereof, 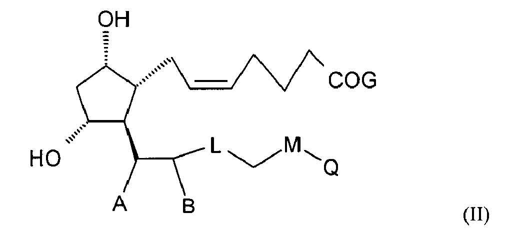 wherein G denotes OH, lower alkoxy, amino-lower-alkyl, ,
A and B are each H or form together an additional bond,
L is CO, CHOR₁ (R₁ being H or lower alkyl),
M is O, S, (CH₂)ₙ, (n being from 1 to 6),
Q is H or Ar, Ar being preferably phenyl which is unsubstituted or substituted one or more times by halogen, hydroxy, lower alkoxy, cyano, halo-lower-alkyl, e.g. trihalomethyl.

3. Combination of claim 1, wherein said benzo[g]quinoline is any compound selected from the group of: 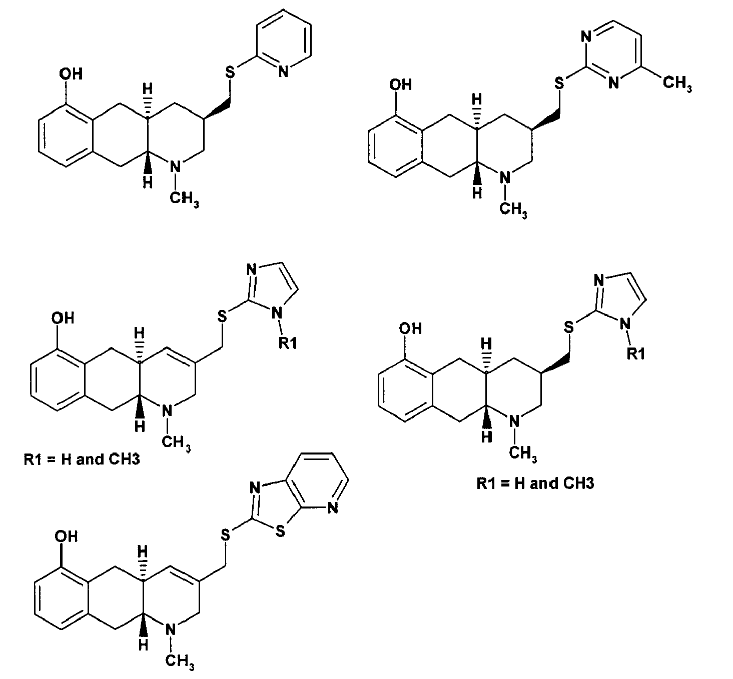 and wherein said prostaglandin is any compound selected from the group of:

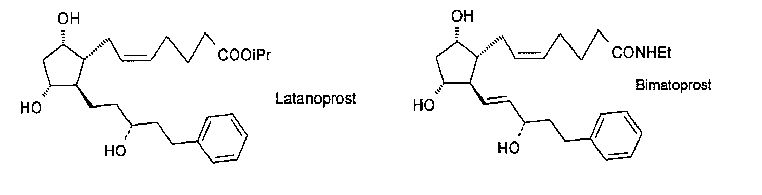

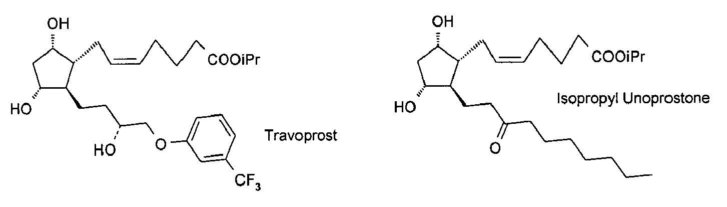

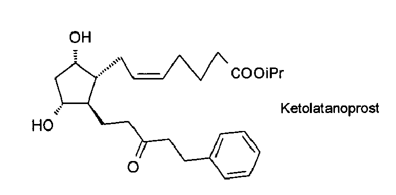

4. A combination of claim 1, wherein said benzo[g]quinoline is a compound of formula (I), and/or a pharmaceutically acceptable salt thereof, A and B are each H or form together an additional bond, 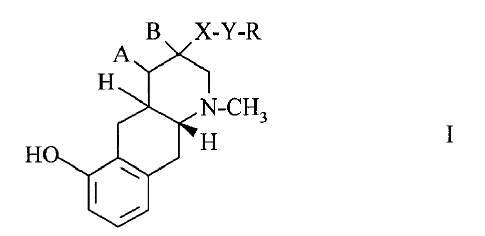 wherein
X is CH₂
Y is S, and
R is of formula (e), (f), or (h), 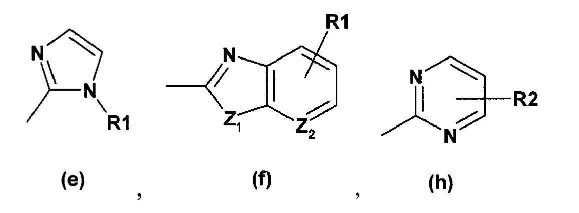
R₁ being H or lower alkyl,
Z₁ being S,
Z₂ being CH or N, and
R₂ being H, or lower alkyl.

5. A combination of claim 4, wherein the prostaglandin is selected from Latanoprost, Travaprost, Bimatoprost and Ketolatanoprost, preferably from Latanoprost and Travaprost, more preferably from Latanoprost.

6. A combination of claim 1 which comprises

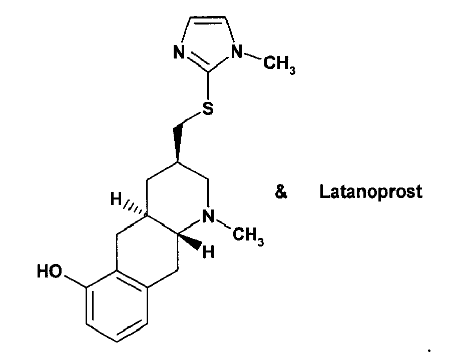

7. A combination of claim 1 which comprises

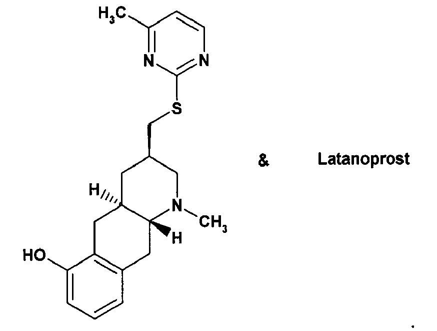

8. A combination of claim 1 which comprises

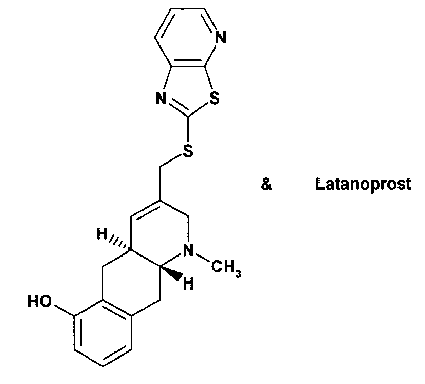

9. Use of a combination in accordance to any of claims 1 - 8 in the manufacture of a medicament for the treatment of glaucoma, e.g. being based upon elevated levels of IOP.

10. Method to treat and or prevent glaucoma in a subject, which method comprises the administration of a medicament to said patient, which medicament comprises a benzo[g]quinoline derivative and a prostaglandin derivative.

11. Method of claim 5 wherein said subject is suffering from elevated levels of IOP.

12. Method of treating normal tension glaucoma (NTG), which method comprises the administration of a medicament to said patient, which medicament comprises a benzo[g]quinoline derivative and a prostaglandin derivative.

13. Method of claim 12, wherein the chorio-retinal and/or optic nerve blood flow of said patient is impaired.

14. Method of claim 10, wherein said administration is topical ocular administration.

15. Combination of claim 1, which is an ophthalmic composition.

16. Combination of claim 15, which is an ophthalmic insert.

17. Combination of claim 1 - 2, wherein the compounds of formula (I) and (II) are present in a ratio of from 50:1 up to 1:50.
